# EUROPÄISCHE PATENTSCHRIFT

(11) **EP 0 999 754 B1**
(45) Veröffentlichungstag und Bekanntmachung des Hinweises auf die Patenterteilung: **18.07.2001**
(21) Anmeldenummer: 99941388.3
(22) Anmeldetag: 22.06.1999
(51) Int. Cl.: A22B 5/00, G01N 33/12, G06T 5/00, G06T 7/40

(54) **VERFAHREN ZUR BEWERTUNG VON SCHLACHTTIERHÄLFTEN DURCH OPTISCHE BILDVERARBEITUNG**
METHOD FOR EVALUATING THE HALVES OF SLAUGHTERED ANIMALS BY OPTICAL IMAGE PROCESSING
PROCEDE D'EVALUATION DE MOITIES D'ANIMAUX ABATTUS PAR TRAITEMENT D'IMAGES OPTIQUE

(30) Priorität: 20.08.1998 DE 19837806
(43) Veröffentlichungstag der Anmeldung: 17.05.2000
(73) Patentinhaber: CSB-System Software-Entwicklung & Unternehmensberatung AG, 52511 Geilenkirchen (DE)
(72) Erfinder: SCHIMITZEK, Dr.Peter, D-52511 Geilenkirchen (DE)
(74) Vertreter: Haussingen, Peter
(86) Internationale Anmeldenummer: DE9901854
(87) Internationale Veröffentlichungsnummer: WO0010396

(56) Entgegenhaltungen:
- WO-A-98/08088
- DE-A- 4 131 556

## Beschreibung

Die Erfindung bezeichnet ein Verfahren zur Bewertung von Schlachttierhälften durch optische Bildverarbeitung, die am Wareneingang, Klassifizierungspunkten oder Warenausgang von Schlacht- und Fleischwarenbetrieben erfaßt werden. Derartige Schlachttierhälften werden in der Regel an einem Hacken hängend mittels spezieller Transportsysteme innerhalb der Schlacht- und Fleischwarenbetriebe befördert. Die vorgestellte Bewertung ist insbesondere für Schlachtschweinehälften ausgelegt, jedoch prinzipiell ebenfalls für Schlachttierhälften von Rindern, Schafen, Ziegen oder andere Groß- und Kleinschlachttieren geeignet.

Im allgemeinen werden die Schlachtschweinehälften registriert, gewogen und bewertet. Die wirtschaftliche Bewertung der Schlachtschweinehälften erfolgt durch eine entsprechende amtliche Handelsklasseneinstufung. Hierbei erfolgt die Erfassung der Speck- und Fleischdicken jeweils landesspezifisch an gesetzlich vorgegebenen Stellen. Zur Sortierung erfolgt in der Regel eine Handelswertbestimmung der Schlachtkörper mit einem höheren Aussagegehalt durch die Einbeziehung einer Vielzahl weiterer spezifischer Parameter, welche jedoch meist nicht standardisiert sind.

Die Druckschriften DE4109345C2 und DE4408604C2 offenbaren eine Analyse bzw. Bewertung von Schlachttierhälften mittels Bildverarbeitung. Bei dieser erfolgt eine photogrammetrische Auswertung von Teilbildern der Schlachttierhälften, wobei vom Kreuzbein ausgegangen wird. Nachteilig bei derartigen Methoden ist, daß das Kreuzbein als markanter Punkt im Bild der Schlachttierhälften unter den üblichen Bedingungen in Schlachtbetrieben nicht hinreichend sicher selektierbar ist, da es auf Grund von gelegentlich auftretenden Spaltfehlern - Teilung erfolgt nicht exakt in der Symmetrieebene des Schlachtkörpers - zu in einer Schlachttierhälfte fehlenden bzw. mit Flomen überdeckten Bereichen der Wirbelsäule kommt, welche bekanntermaßen nur in einem schmalen Bereich der Symmetrieebene des Tierkörpers angeordnet ist. Ein weiterer Nachteil ist die aufwendige und rechenintensive Selektion der Wirbelsäule über eine Objektanalyse mit vordefinierten Kontur- und Objektparametern.

In der Druckschrift WO 98/08088 A1 wird ein Verfahren und eine Vorrichtung zur Bestimmung von charakteristischen Fleisch- und Rumpfparametern von Schlachttieren bei Benutzung der Bildanalyse offenbart, wobei zunächst der äußere Umriss des Rumpfs mit einer Vielzahl darauf angeordneter, anatomische Merkmale repräsentierende, erster Referenzpunkte, die an Hand von hervortretenden oder markanten Punkten bestimmt werden, identifiziert wird. In weiteren Schritten werden an vorbestimmten Stellen zweite Referenzpunkte relativ zu den Ersten auf oder innerhalb der Umrisslinie des Rumpfs bestimmt, die zur Aufteilung des Rumpfs in eine Vielzahl von Abschnitten genutzt werden.
Für die Berechnung des Einstufungsparameters wird eine Wahrscheinlichkeitsgleichung benutzt, bei der der Einstufungsparameter als abhängige Variable enthalten ist und ein Gebiet, das mit den ersten und zweiten markanten Punkten bestimmt ist, als unabhängige Variable. Die eigentliche Einstufung des Schlachttiers erfolgt im weiteren Verlauf des Zerlegungsprozesses, nachdem ein transversaler Schnitt zwischen der 12. und 13. Rippe erfolgt ist, indem diese auf der Basis des Querschnitts des Rückenmuskels (musculus longissimus dorsi)sowie des enthaltenen intramuskulären Fetts erfolgt. Nachteilig an diesem Verfahren ist die Ermittlung einer Vielzahl von Referenzpunkten, die einen erheblichen rechentechnischen Selektionsaufwand erfordern. Der zusätzlich Aufwand für den notwendigen Schnitt zwischen den Rippen zur Beurteilung des Rückenmuskels steigert die Kosten. Die Anwendung dieses Verfahrens wird insbesondere bei speziellen Selektionsanforderungen erfolgen.

Aufgabe der Erfindung ist es, ein Verfahren zu entwickeln, daß die Bewertung von Schlachttierhälften mittels Bildbearbeitung unter Beseitigung obiger Nachteile absichert, wobei insbesondere mit einem einfachen rechentechnisch zu realisierenden Verfahren eine hinreichend sichere Bewertung erfolgen soll, auch wenn durch Spaltfehler die Symmetrieebene bei der Spaltung der Schlachttiere verlassen wird.

Die Aufgabe wird mit den im Patentanspruch 1 genannten Merkmalen gelöst. Weiterbildungen der Erfindung ergeben sich aus den Unteransprüchen.

Die Aufgabe wird im wesentlichen dadurch gelöst, daß zur photogrammetrischen Auswertung und Bewertung nur diejenigen Körperbestandteile verwendet werden, die sich anatomisch über einen breiten Bereich zur Symmetrieebene des Schlachttierkörpers erstrecken und optisch sicher erfaßbar sind, wobei insbesondere der Schloßknochen, der Musculus-Gluteus-Medius (MGM) und der Rückenspeck diese Anforderungen erfüllt.

Durch die Existenz zweier Schloßknochen, welche sich lediglich in der Symmetrieebene (innerhalb des Hüftgelenkes) treffen, verläuft der Schnitt stets durch einen Schloßknochen und ist somit optisch sichtbar und rechentechnisch sicher auswertbar. Da dieser nicht von Flomenfett überlappt werden kann, ist eine optische Verdeckung der Spaltfläche nicht möglich. Der MGM und der Rückenspeck verlaufen gleichartig über weite Teile der Rückenbreite und sind über die Helligkeits- bzw. Farbübergänge stets sicher optisch und rechentechnisch selektierbar.

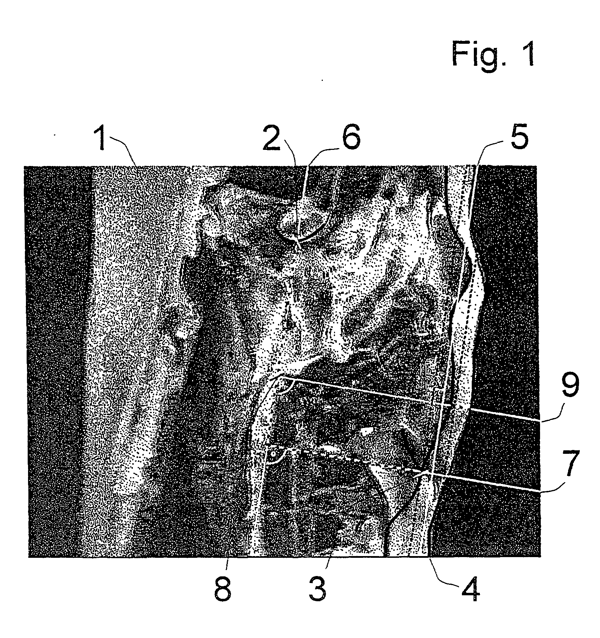

Ein vorteilhaftes Ausführungsbeispiel der Erfindung wird in Fig. 1 als photogrammetrischen Auswertung näher erläutert.

Nach Fig. 1 beinhaltet ein zur photogrammetrischen Auswertung bereitgestellter Bildbereich 1 eine optische Aufnahme der Lenden- und Schinkenregion der Schlachttierhälfte, inclusive des körperseitigen Endes des Schloßknochens 2, des körperseitigen Endes des MGM 3 und der äußeren Grenze des Rückenspecks 4. Der oval rundliche, auf Grund der Helligkeits- bzw. Farbübergänge gut sichtbare und rechentechnisch selektierbare Schloßknochen (hell vor rotem Randbereich) wird erkannt und vorteilhaft das körperseitige Ende des Schloßknochen 2 als erster markanter Punkt der photogrammetrischen Auswertung zur Verfügung gestellt. Des weiteren wird der Rückenspeck bezüglich seiner Grenzen auf Grund der Helligkeits- bzw. Farbübergänge (hell vor rotem Randbereich bzw. dem Hintergrund) erkannt und hinsichtlich seines gemittelten Verlaufs als eine erste Gerade 5 der photogrammetrischen Auswertung zur Verfügung gestellt. Denkbar ist, gleichzeitig dessen Grenzen, das Flächenmaß oder die Breite zu bestimmen oder zu erfassen und eine Stelle minimaler Speckdicke als zweiten markanten Punkt der photogrammetrischen Auswertung zur Verfügung zu stellen. Es ist jedoch vorteilhaft, den MGM 3 auf Grund der Helligkeits- bzw. Farbübergänge (rot vor hellem Randbereich) zu erkennen und das körperseitige Ende des MGM 3 als zweiten markanten Punkt der hotogrammetrischen Auswertung zur Verfügung zu stellen. Bei vorbekannter Seite der Schlachttierhälfte läßt sich rechentechnisch die Gerade 5 als Parallele 6 durch den ersten markanten Punkt - in Form des körperseitigen Endes des Schloßknochens 2 - darstellen, worauf durch den zweiten Punkt eindeutig eine Senkrechte 7 bestimmt ist, welche vorteilhaft bezüglich seiner Teilstrecken bis zur Parallelen 6 und der äußeren Grenze des Rückenspecks 4 berechnet wird. Eine vorteilhafte Weiterbildung stellt die Berechnung der Länge vom ersten markanten Punkt - in Form des körperseitigen Endes des Schloßknochens 2 - zum Fußpunkt der Senkrechten 7 dar, wobei von dieser Länge abgeleitete weitere Fußpunkte 8 berechnet werden können - bspw. ein Punkt, der diese Linie in einem Verhältnis 2 zu 1 teilt, durch den weitere Senkrechte 9 bestimmt sind und deren Teilstrecken vorteilhaft analog berechnet werden. Die berechneten Längen der Teilstrecken bzw. Flächenwerte zwischen diesen dienen als spezifische Parameter zur Bewertung der Schlachttierhälfte. Es ist insbesondere vorteilhaft über diese rechentechnisch sichere Selektion von zwei Punkten und einer Richtung, die klassische Zwei-Punkt-Methode in modifizierter Form photogrammetrisch nachzubilden.

### Verwendete Bezugszeichen

- 1: Bildbereich
- 2: körperseitigen Endes eines Schloßknochens
- 3: körperseitigen Endes des MGM
- 4: Rückenspecks
- 5: Gerade
- 6: Parallele
- 7: Senkrechte
- 8: weitere Fußpunkte
- 9: weitere Senkrechte

## Patentansprüche

1. Verfahren zur Bewertung von Schlachttierhälften durch optische Bildverarbeitung, wobei ein zur photogrammetrischen Auswertung bereitgestellter Bildbereich (1) eine optische Aufnahme der Lenden- und Schinkenregion der Schlachttierhälfte beinhaltet und gut sichtbare Körperbestandteile rechentechnisch erkannt und spezifische Parameter zur Bewertung ermittelt werden, indem zwei markante Punkte und eine Gerade (5) photogrammetrisch sicher erfaßt werden, dadurch gekennzeichnet,
- daß als ein erster markanter Punkt das körperseitige Ende eines Schloßknochens (2) und als Gerade (5) der mittlere Verlauf des Rückenspecks (4) benutzt werden,
- daß zur Geraden (5) eine Parallele (6) durch den ersten markanten Punkt sowie eine Senkrechte (7) durch den zweiten markanten Punkt berechnet werden und
- daß die Längen der Teilstrecken der Senkrechten (7) zur Parallelen (6) und zur äußeren Grenze des Rückenspecks (4) als spezifische Parameter zur Bewertung dienen.

2. Verfahren zur Bewertung von Schlachttierhälften durch optische Bildverarbeitung nach Anspruch 1, dadurch gekennzeichnet,
daß als zweiter markanter Punkt die Stelle der minimalen Speckdicke des Rückenspecks (4) oder das körperseitige Ende des MGM (3) photogrammetrisch sicher erfaßt wird.

3. Verfahren zur Bewertung von Schlachttierhälften durch optische Bildverarbeitung nach Anspruch 1 oder Anspruch 2,
dadurch gekennzeichnet,
daß die Länge der Strecke auf der Parallelen (6) vom ersten markanten Punkt zum Fußpunkt der Senkrechten (7) berechnet wird.

4. Verfahren zur Bewertung von Schlachttierhälften durch optische Bildverarbeitung nach Anspruch 3, dadurch gekennzeichnet,
daß von der berechneten Länge der Strecke weitere Fußpunkte (8) auf der Parallelen (6) und weitere Senkrechte (9) berechnet werden.

5. Verfahren zur Bewertung von Schlachttierhälften durch optische Bildverarbeitung nach einem der Ansprüche 1 bis 4,
dadurch gekennzeichnet,
daß eine zur sicheren photogrammetrischen Erfassung modifizierte Zwei-Punkte-Methode realisiert wird.

## Claims

1. Method for evaluating slaughtered animal halves by optical image processing, wherein an image region (1) provided for photogrammetric evaluation contains an optical reproduction of the loin and hindquarters region of the slaughtered animal half and uses computer means to distinguish clearly visible body parts, and specific parameters are determined for evaluation, wherein two defined points and a straight line (5) are reliably determined photogrammetrically, characterised in that,
- the body-side end of a hip-joint bone (2) is used as a first defined point and the middle course of the back flesh (4) is used as the straight line (5),
- with respect to the straight line (5), a parallel (6) through the first defined point and a perpendicular (7) through the second defined point are calculated and
- the lengths of the partial sections of the perpendiculars (7) with respect to the parallel (6) and with respect to the outer limit of the back flesh (4) serve as specific parameters for the evaluation.

2. Method for evaluating slaughtered animal halves by optical image processing according to claim 1, characterised in that,
as the second defined point, the location of the minimum fat thickness of the back flesh (4) or the body-side end of the MGM (3) is reliably determined photogrammetrically.

3. Method for evaluating slaughtered animal halves by optical image processing according to claim 1 or claim 2, characterised in that,
the length of the section on the parallel (6) from the first defined point to the foot of the perpendiculars (7) is calculated.

4. Method for evaluating slaughtered animal halves by optical image processing according to claim 3, characterised in that,
from the calculated length of the section, further feet (8) on the parallel (6) and further perpendiculars (9) are calculated.

5. Method for evaluating slaughtered animal halves by optical image processing according to one of claims 1 to 4, characterised in that a two-point method modified for reliable photogrammetric determination is produced

## Revendications

1. Procédé d'exploitation de demi-carcasses d'animaux par traitement optique de l'image, selon lequel une zone d'image (1), préparée pour l'exploitation photogrammétrique, comporte une prise de vue optique de la région lombaire et d'échine de la demi-carcasse, on reconnaît par le calcul des parties de corps bien visibles, et on détermine des paramètres spécifiques pour l'exploitation par la saisie photogrammétrique certaine de deux points marquants et d'une droite (5), caractérisé en ce qu',
- on utilise, comme premier point marquant, l'extrémité intérieure de l'os d'attache (2) et comme droite (5), le tracé moyen du gras du dos (4),
- on calcule une parallèle (6) à la droite (5) passant par le premier point marquant ainsi qu'une perpendiculaire (7) à cette droite, passant par le second point marquant, et
- les longueurs des parties de tracé de la perpendiculaire (7) à la parallèle (6) et jusqu'à la limite extérieure du gras du dos (4) servent comme paramètres spécifiques pour l'exploitation.

2. Procédé d'exploitation de demi-carcasses d'animaux abattus, par traitement optique de l'image selon la revendication 1, caractérisé en ce que,
comme second point marquant on saisit par voie photogrammétrique le point de l'épaisseur minimale du gras du dos (4) ou l'extrémité du côté du corps du point MGM (3).

3. Procédé d'exploitation de demi-carcasses d'animaux abattus par traitement optique de l'image selon la revendication 1 ou la revendication 2, caractérisé en ce qu',
on calcule la longueur du chemin sur la parallèle (6), entre le premier point marquant et le pied de la perpendiculaire (7).

4. Procédé d'exploitation de demi-carcasses d'animaux abattus par traitement optique de l'image selon la revendication 3,caractérisé en ce qu',
à partir de la longueur calculée du chemin on calcule d'autres pieds (8) sur la parallèle (6) et d'autres perpendiculaires (9).

5. Procédé d'exploitation de demi-carcasses d'animaux abattus par traitement optique de l'image selon la revendication 3,caractérisé en ce que,
pour garantir la saisie photogrammétrique on réalise une méthode à deux points modifiée.
